# EUROPÄISCHE PATENTSCHRIFT

(11) **EP 1 002 502 B1**
(45) Veröffentlichungstag und Bekanntmachung des Hinweises auf die Patenterteilung: **31.03.2004**
(21) Anmeldenummer: 99122880.0
(22) Anmeldetag: 18.11.1999
(51) Int. Cl.: A61B 19/00

(54) **Vorrichtung zur Unterstützung des Positionierens und Ausrichtens eines manuell frei bewegbaren Werkzeuges**
Apparatus for assisting the positioning and alignment of a manually freely movable tool
Dispositif pour assister le positionnement et l'alignement d'un outil à mouvement libre manuellement

(30) Priorität: 18.11.1998 EP 98121887
(43) Veröffentlichungstag der Anmeldung: 24.05.2000
(73) Patentinhaber: Fraunhofer-Gesellschaft zur Förderung der angewandten Forschung e.V., 80686 München (DE)
(72) Erfinder: Wisskirchen, Peter, Dr., 53359 Rheinbach (DE); Grunst, Gernot, Dr., 53773 Hennef/Sieg (DE)
(74) Vertreter: Hilleringmann, Jochen, Dipl.-Ing.

(56) Entgegenhaltungen:
- EP-A- 0 427 358
- EP-A- 0 830 847
- DE-A- 4 213 426
- US-A- 5 526 814

## Beschreibung

Die Erfindung betrifft eine Vorrichtung zur Unterstützung des Positionierens und Ausrichtens eines manuell frei bewegbaren, eine Längsachse aufweisenden Werkzeuges relativ zu einem zu bearbeitenden Objekt. Anwendung findet diese Vorrichtung insbesondere in der bildgeführten Chirurgie, und zwar in der präoperativen Phase und in der Phase bis unmittelbar vor dem Eingriff und gegebenenfalls auch während des Eingriffs.

Die bildgeführte Chirurgie ist ein neuartiger Ansatz zur Verbesserung der Sicherheit und Wirksamkeit von chirurgischen Eingriffen. Diese Technik verwendet präoperativ gewonnene Bilder, wie Ultraschall, Computer-Tomographie (CT) oder Magnetic Resonance Imaging (MRI). Diese Informationen werden für die Therapieplanung und die intraoperative Kontrolle der Position von chirurgischen Instrumenten verwendet. Darüber hinaus können diese Systeme dazu beitragen, Instrumente bis zum Zielgewebe, z.B. einem Gehimtumor, zu führen. Beispiele für derartige Systeme sind in EP-A-0 427 358 und EP-A-0 830 847 beschrieben.

Das in EP-A-0 427 358 beschriebene System betrifft eine Vorrichtung zur Unterstützung des Positionierens und Ausrichtens eines manuell frei bewegbaren, eine Längsachse aufweisenden Werkzeuges relativ zu einem zu behandelnden Objekt. Das bekannte System umfasst eine Verarbeitungseinheit zum Speichern und Verarbeiten von Volumenbildelementen, eine Anzeigevorrichtung zum Anzeigen der gespeicherten Volumenbildelemente und einen Detektor für das Werkzeug zur Detektion der Position und Ausrichtung des Werkzeuges reiativ zum Objekt und zum Liefern von Positions- und Ausrichtungsdaten an die Verarbeitungseinheit, wobei die Anzeigevorrichtung von dieser derart ansteuerbar ist, dass eine Repräsentation des Werkzeuges mit anzeigbar ist. Das bekannte System umfasst ferner eine Dateneingabevorrichtung zum Markieren einer Eintrittsstelle.

Durch den Eingriff können anatomische Veränderungen hervorgerufen werden, wodurch die vorher gewonnenen, d.h. präoperativen Bilddaten ungültig werden. In einer solchen Situation ist ein intraoperatives Nachbessern der Daten notwendig. Dies können interventionelle MRI-Geräte leisten. Im Gegensatz zu präoperativen Tomogrammen (dreidimensionale Datensätze von vom Innern des Objekts aufgenommenen Volumenbildelementen), die das ganze interessierende Volumen in relativ guter Qualität erfassen und ohne Verzögerung jede Ansicht bzw. Schnittebene anzeigen können, leiden interventionell erstellte Tomogramme darunter, dass lediglich nur Schichtbilder in vergleichsweise schlechter Qualität und mit zeitlicher Verzögerung (von einigen Sekunden) erzeugt werden können. Bei der interventionellen Tomographie werden dem Operateur lediglich Schnittbilder gezeigt, die die Geräteebene ohne Kontext und Zielstrukturen zeigen. Der Bildaufbau dauert im allgemeinen etwa 5 Sekunden oder mehr. Ohne Orientierungshilfen ist es für den Operateur äußerst schwierig, ausgehend von einer am zu bearbeitenden Objekt, beispielsweise Kopf, festgelegten Eintrittsstelle mit dem chirurgischen Instrument bis zur Bearbeitungsstelle (Zielstruktur) zu gelangen.

Der Erfindung liegt die Aufgabe zugrunde, eine Vorrichtung zu schaffen, die die Positionierung und Ausrichtung eines manuell frei bewegbaren Werkzeuges relativ zu einem zu bearbeitenden Objekt durch kognitiv adäquate Bildgebung visuell unterstützt.

Zur Lösung dieser Aufgabe wird mit der Erfindung eine Vorrichtung nach Ansprüch 1, vorgeschlagen, mit der die Positionierung und Ausrichtung eines manuell frei bewegbaren, eine Längsachse aufweisenden Werkzeuges relativ zu einem zu bearbeitenden Objekt unterstützt wird; die Merkmale einzelner Weiterbildungen der Erfindung sind in den Unteransprüchen angegeben.

Nach der Erfindung wird ein Leitbild erstellt, das aufgrund von vor der Bearbeitung gewonnener Volumenbildelemente erzeugt wird und in dem das Werkzeug in der Projektion bei Betrachtung der Bildebene mit angezeigt wird. Auf dem Leitbild ist die auf der Oberfläche des Objekts liegende Eintrittsstelle markiert. Die in dem Leitbild gezeigte Schnittebene (nachfolgend auch Eintrittsstellen-Schnittebene genannt) verläuft senkrecht zur Verbindungslinie zwischen der Eintrittsstelle und der Bearbeitungsstelle, die ihrerseits im Innern des Objekts liegt und zuvor bei Betrachtung der Volumenbildelemente und der sich daraus ergebenden unterschiedlichen Schnittebenen festgelegt worden ist. Anhand der Koordinaten der Bearbeitungsstelle und der Eintrittsstelle kann diese Verbindungslinie errechnet werden, so dass dann anhand der vor der Bearbeitung gewonnenen Volumenbildelemente diejenige Schnittebene dargestellt werden kann, die in dem gewünschten Winkel zur Verbindungslinie verläuft und die Eintrittsstelle beinhaltet. Das Werkzeug ist mit einem Detektor zur Erfassung der Werkzeugposition und -ausrichtung relativ zum zu behandelnden Objekt versehen. Aufgrund dieser Detektion ist es möglich, das Werkzeug nun im Leitbild vor der Eintrittsstellen-Schnittebene entsprechend seiner aktuellen Positionierung und Ausrichtung in der Projektion darzustellen. Aufgrund dieser Visualisierung ist es nun für den Bearbeiter möglich, das manuell bewegbare Werkzeug so auszurichten, dass es eine vorgebbare Lage relativ zum Objekt aufweist. Insbesondere ist es möglich, die Längsachse des Werkzeuges mit der Verbindungslinie zwischen Bearbeitungsstelle und Eintrittsstelle fluchtend auszurichten. Da die Eintrittsstellen-Schnittebene selbst senkrecht zur Verbindungslinie verläuft, reduziert sich die Darstellung des Werkzeuges im Leitbild zu einem Punkt, da das Werkzeug in der Projektion und damit in Richtung seiner Längsachse dargestellt ist.

Durch die Bereitstellung von intuitiven visuellen Orientierungshilfen für den Bearbeiter wird mit Hilfe der erfindungsgemäßen Vorrichtung eine präzise und zügige Positionierung von Werkzeugen unterstützt. Das Leitbild dient als eine Art Zielvorrichtung und unterstützt damit die Positionierung und Ausrichtung des Werkzeuges relativ zum zu behandelnden Objekt.

In vorteilhafter Weiterbildung der Erfindung ist vorgesehen, dass auf dem Leitbild auf eine Darstellung des Objekts mit Werkzeug umgeschaltet werden kann, die ebenfalls anhand von zuvor gewonnenen Volumenbildelementen erzeugt wird und die eine die Verbindungslinie zwischen der Behandlungsstelle und der Eintrittsstelle beinhaltende Schnittebene zeigt. Durch diese Umschaltung ist es beispielsweise möglich, die Eindringtiefe des Werkzeuges von der Eintrittsstelle aus in das Objekt zu visualisieren.

Nach der Erfindung wird also zur Positions- und Ausrichtungsunterstützung auf vor der Bearbeitung gewonnene Datensätze von Volumenbildelementen zurückgegriffen, die jeweils als Leitbild angezeigt werden. Da die zuvor gewonnenen Datensätze nicht notwendigerweise den aktuell bei der Behandlung vorzufindenden Gegebenheiten des Objekts entsprechen müssen, ist es zweckmäßig, zusätzlich ein Kontrollbild anzuzeigen, das aufgrund aktuell gewonnener Volumenbildelemente erzeugt wird und Schnittbilder zeigt, die in einer vorbestimmten einstellbaren Ausrichtung relativ zum Werkzeug verlaufen. Während die Vorpositionierung und Vorausrichtung anhand der zuvor gewonnenen Volumenbildelemente realisiert wird, wird das Einführen des Werkzeuges in das Objekt optisch über das Kontrollbild kontrolliert, wobei die Positionierung und Ausrichtung beim Einführen des Werkzeuges wiederum anhand des Leitbildes unterstützt werden kann.

Nachfolgend wird anhand der Zeichnung die Erfindung bei Anwendung als stereotaktisches Bildleitverfahren für Eingriffe mit interventionellen Tomographen unter intraoperativer Bildkontrolle näher erläutert. Im einzelnen zeigen:
- Fig. 1: eine schematische Darstellung des Systems für das stereotaktische Bildleitverfahren für Eingriffe mit interventionellen Tomographen unter intraoperativer Bildkontrolle,
- Fig. 2: schematisch die geometrischen Elemente, die zur Ausrichtung benötigt werden, nämlich Eintrittsstelle, Zielstelle oder Behandlungsstelle und Instrument, in einem integrierten Szenario und
- Fign. 3 bis 5: verschiedene Darstellungen von Leitbildern zum Ausrichten des Operationsinstruments bei beliebiger Ausgangsposition (Fig. 3), bei korrekt an der Eintrittsstelle positionierter Spitze des Instruments (Fig. 4) und bei fluchtender Ausrichtung der Achse des Instruments mit der Verbindungslinie zwischen der Eintrittsstelle und der Ziel- bzw. Bearbeitungsstelle.

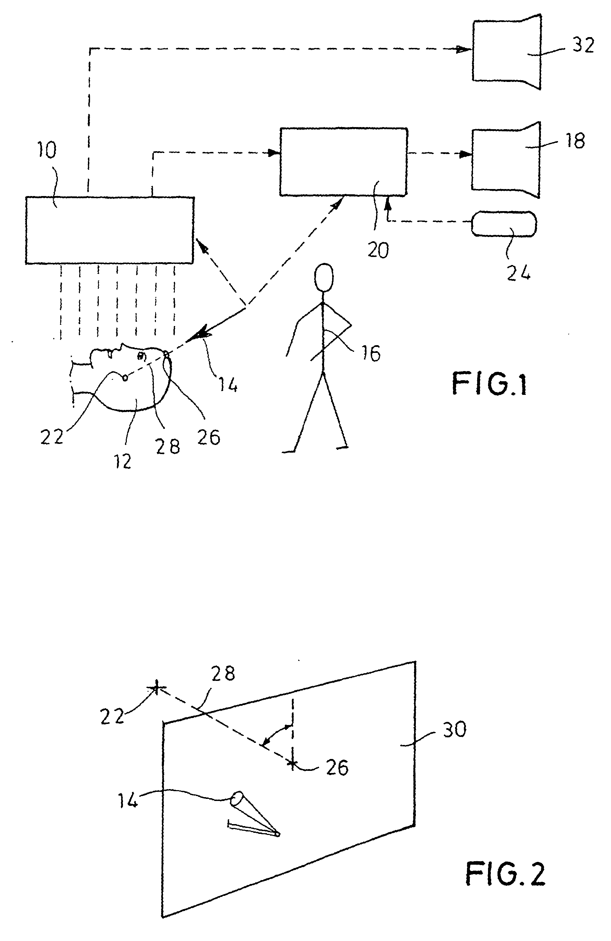

Die Erfindung wird besonders deutlich, wenn man sich ihre Anwendung bei einem Tomographen vor Augen führt. Das sich dadurch ergebende Gesamtsystem ist schematisch in Fig. 1 gezeigt.

Tomographen erstellen dreidimensionale Datensätze (Tomogramme), die das Innere von geschlossenen Objekten zeigen. Damit werden minimalinvasive Eingriffe auf innere Zielstrukturen möglich, ohne das Objekt selbst signifikant zu zerstören. Ein Beispiel ist die minimalinvasive Behandlung von Gehirntumoren mit Hilfe von Magnetresonanz-Tomographen.

Interventionelle Tomographen 10 erlauben es, zusätzlich zu einem präoperativ erhobenen Tomogramm, während des Eingriffs laufend Schnittebenen durch das Objekt 12 zu erheben, die den aktuellen Zustand anzeigen. Die Position der Schnittebenen wird relativ zur aktuellen Position eines mit einem Positions- und Ausrichtungsdetektor 13 versehenen Operationsgeräts (bei 14 angedeutet) festgelegt. Damit können auch Eingriffe durchgeführt und kontrolliert werden, bei denen sich das Innere des Objekts 12 durch den Eingriff verformen kann.

Das nachfolgende Ausführungsbeispiel beschreibt ein stereotaktisches Bildleitverfahren, das intraoperativ erhobene Schnittebenen mit dem präoperativ erhobenen Tomogramm und synthetischen Steuerungshilfen integriert, so dass während des Eingriffs die Positionierung, Ausrichtung und Bewegung des Operationsgerätes 14 (z.B. ein Katheter) schnell, sicher und ergonomisch optimal ausgeführt und kontrolliert werden können.

Das Bildleitverfahren für Eingriffe im interventionellen Tomographen unter intraoperativer Bildkontrolle läuft nach folgendem Schema ab.

Zunächst wird das Untersuchungsobjekt 12 in den interventionellen Tomographen eingebracht und ein Tomogramm erhoben. Anschließend wird im Tomogramm das Ziel markiert. Dazu visualisiert der Operateur 16 (ebenenweise oder als Volumen) das Tomogramm im Leitbild-Monitor 18 des Bildleitsystem-Computers 20, bis er interaktiv die Zielposition 22 im Tomogramm gefunden hat. Eine über ein Dateneingabegerät 24 eingegebene Markierung im dreidimensionalen Volumen markiert die gefundene Zielposition 22.

Danach markiert der Operateur 16 die Eintrittsstelle 26. Hierbei visualisiert der Operateur 16 (ebenenweise oder als Volumen) das Tomogramm im Leitbild-Monitor 18 des Bildleitsystem-Computers 20, bis er interaktiv eine geeignete Eintrittsposition 26 gefunden hat. Alternativ kann der Eintritt auch am realen Objekt mit einem Positionsgeber identifiziert werden. Eine Markierung im dreidimensionalen Volumen markiert die Eintrittsposition 26. Die Eintrittsstelle wird am Objekt 12 geöffnet.

Nun kann die Ausrichtung des Operationsinstruments mit Hilfe eines Leitbildes erfolgen. Das Operationsinstrument 14 muss am realen Objekt 12 so ausgerichtet werden, dass es an der markierten Eintrittsstelle 26 in das Objekt 12 eintritt und im dreidimensionalen Raum exakt in Richtung auf das Ziel 22 hin geführt wird. Die Positionierung und Ausrichtung des Instruments 14 ist in aktuellen interventionellen Systemen ein Problem und eine sehr aufwendige und komplizierte Aktion, weil dem Operateur 16 keine geeigneten Orientierungshilfen zur Verfügung gestellt werden. Es wird lediglich ein zweidimensionales Schnittbild parallel oder senkrecht zum Instrument 14 angeboten, das zudem erst verzögert eintrifft.

Bei der Erfindung werden die relevanten Informationen über die Lage von Eintritt 26, Ziel 22 und Instrument 14 in einer Weise illustriert, dass der Operateur 16 unmittelbar sieht, wie er das angestrebte Ziel erreichen kann. Ein schematischer Überblick ist in Fig. 2 gezeigt.

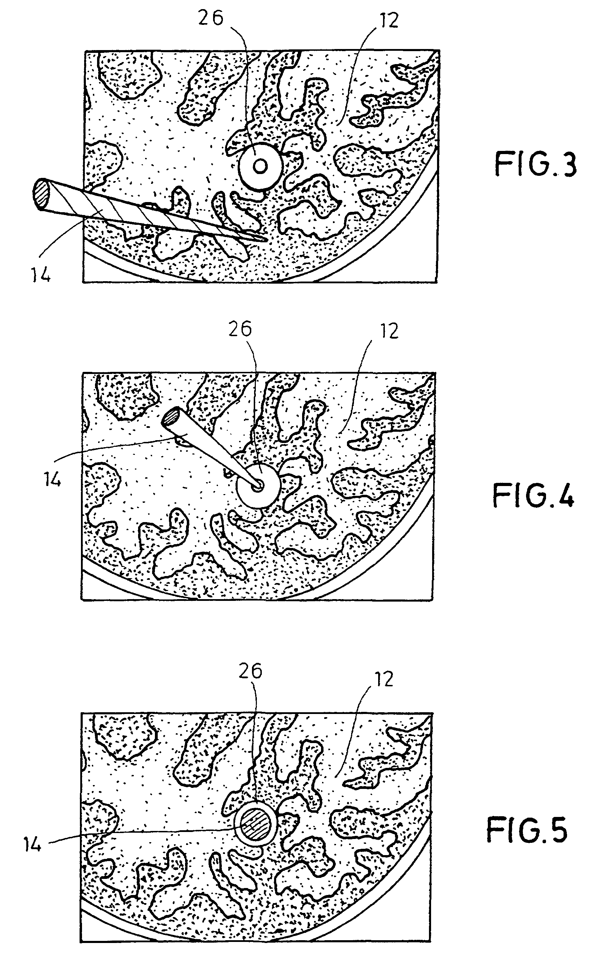

Aufgrund der im Tomogramm markierten Positionen für Eintritt 26 und Ziel 22 und der aktuell gemessenen Position und Ausrichtung des Operationsinstruments 14 kann das Bildleitsystem ein reduziertes Situationsbild anbieten, das die kognitivergonomische Integration der zwei- und dreidimensionalen Bilddaten ist, die für die Ausrichtungsaufgabe benötigt werden. Das Situationsbild enthält folgende Elemente (siehe Fign. 3 bis 5):
- Schnittebene durch den Eintritt senkrecht zur Verbindungslinie 28 (siehe Fign. 1 und 2) von Eintritt 26 zum Ziel 22 (Projektionsebene 30).
- Markierung des Eintritts 26 im Schnittbild. Das Ziel liegt im 3D-Raum senkrecht unter diesem Punkt.
- Die Projektion des Operationsinstruments 14 in aktuell gemessener Position und Ausrichtung.

Fig. 3 zeigt eine noch nicht optimale Ausrichtung und Positionierung des Operationsinstruments 14 relativ zur Eintrittsstelle 26. Das Operationsinstrument 14 ist optimal positioniert, wenn die Spitze in der Eintrittsmarkierung 26 liegt (Fign. 4 und 5) und optimal ausgerichtet, wenn seine Projektion zu einem Kreis wird (Fig. 5).

Anschließend kann die Einführung des Operationsinstruments 14 unter intraoperativer Bildkontrolle erfolgen. Dabei kann die durch das Leitbild des Monitors 18 erreichte Positionierung des Operationsinstruments 14 auf Korrektheit kontrolliert werden, wenn durch die intraoperative Bildgebung auf dem Kontrollbild-Monitor 32 Schnittbilder parallel und senkrecht zum Operationsinstrument 14 erhoben werden. Es kann visuell geprüft werden, ob die aus dem präoperativen Tomogramm gefundene Position korrekt ist, und gegebenenfalls können kleine Korrekturen ausgeführt werden. Die Prüfung wird erfindungsgemäß vereinfacht durch gleichzeitige Anzeige der den aktuellen Bildern des Kontrollbild-Monitors 32 entsprechenden Bilder aus dem präoperativen Tomogramm auf dem Leitbild-Monitor 32, die im allgemeinen eine bessere Bildauflösung haben und zudem Markierungen für die intendierten Eintritts- und Zielpunkte 26,22 haben.

Beim Einführen des Operationsinstruments 14 kann diese Prüfung kontinuierlich fortgesetzt werden. Dabei geben die Schnittbilder aus dem präoperativen Tomogramm auf dem Leitbild-Monitor 18 ein schnelles Feedback, während die langsameren und qualitativ schlechteren intraoperativen Bilder auf dem Kontrollbild-Monitor 32 zur Verifikation der Prozedur an kritischen Stellen hinzugezogen werden.

## Patentansprüche

1. Vorrichtung zur Unterstützung des Positionierens und Ausrichtens eines manuell frei bewegbaren, eine Längsachse aufweisenden Werkzeuges retativ zu einem zu behandelnden Objekt, mit
- einer Verarbeitungseinheit (20) zum Speichern und Verarbeiten von Volumenbildelementen vom Innern des Objekts (12)
- einer Anzeigevorrichtung (18), zum Anzeigen der gespeicherten Volumenbildelemente,
- einer Daterieingabevorrichtung (24) zum Markieren einer Bearbeitungsstelle (22) im Innern des Objekts (12) und zum Markieren einer Eintrittsstelle (26) auf der Oberseite des Objekts (12) zum Einführen des Werkzeuges (14) und als Zugang zur Bearbeitungsstelle (22) durch das Werkzeug (14), wobei die Bearbeitungs- und die Eintrittsstelle (22,26) anhand der von der Anzeigevorrichtung (18) angezeigten gespeicherten Volumenbildelementen des Objekts (12) markierbar und in der Verarbeitungseinheit (20) speicherbar sind, und
- einem Detektor (13) für das Werkzeug (14) zur Detektion der Position und Ausrichtung des Werkzeuges (14) relativ zum Objekt, und zum Liefern von Positions- und Ausrichtungsdaten an die Verarbeitungseinheit (20), wobei
- von der Verarbeitungseinheit (20) die Anzeigevorrichtung (18) derart ansteuerbar ist, dass
- auf der Basis der gespeicherten Volumenbildelemente und der gespeicherten Eintrittsstelle diese sowie diejenige Eintrittsstellen-Schnittebene des Objekts (12), die senkrecht zur Verbindungslinie (28) zwischen der Bearbeitungsstelle (22) und der Eintrittsstelle (26) verläuft und die Eintrittsstelle (26) beinhaltet, darstellbar sind,
- und dass in dieser Darstellung der Eintrittsstellen-Schnittebene auch eine Repräsentation des Werkzeuges (14) mit anzeigbar ist, und zwar als Projektion bei Betrachtung in Richtung auf die Eintrittsstellen-Schnittebene und vor dieser angeordnet, so dass das Werkzeug (14) anhand der Darstellung der Eintrittsstellen-Schnittebene als Leitbild mit davor dargestellter Werkzeugprojektion so lange positioniert und ausgerichtet werden kann, bis die Längsachse des Werkzeuges (14) mit der Verbindungslinie (28) zwischen der Bearbeitungsstelle (22) und der Eintrittsstelle (26) fluchtet.

2. Vorrichtung nach Anspruch 1, **dadurch gekennzeichnet, dass** die Anzeigevorrichtung (18) von der Verarbeitungseinheit (20) derart ansteuerbar ist, dass das Objekt (12) mit der Repräsentation des Werkzeuges (14) darstellbar ist, die anhand der zuvor aufgenommenen Volumenbildelemente erzeugt wird und die eine die Verbindungslinie (28) zwischen der Bearbeitungsstelle (22) und der Eintrittsstelle (26) beinhaltende Schnittebene zeigt.

3. Vorrichtung nach Anspruch 1 oder 2, **dadurch gekennzeichnet, dass** eine Vorrichtung (10) zur Erzeugung von Volumenbildelementen des Objekts (12) vorgesehen ist und dass die Anzeigevorrichtung (18) von der Verarbeitungseinheit (20) derart ansteuerbar ist, dass beim Eindringen des Werkzeuges (14) in das Objekt (12) insbesondere zusätzlich eine Darstellung von Schnittebenen als Kontrollbild gezeigt wird, die vorbestimmte einstellbare Ausrichtungen relativ zum Werkzeug (14) aufweisen und anhand von aktuell vom Objekt (12) aufgenommenen Volumenbildelementen erzeugt werden.

4. Vorrichtung nach einem der Ansprüche 1 bis 3, **dadurch gekennzeichnet, dass** die Anzeigevorrichtung (18) von der Verarbeitungseinheit (20) derart ansteuerbar ist, dass beim Eindringen des Werkzeuges (14) in das Objekt (12) insbesondere zusätzlich eine Darstellung von Schnittebenen als Leitbild gezeigt wird, die vorbestimmte einstellbare Ausrichtungen relativ zum Werkzeug (14) aufweisen und anhand der zuvor vom Objekt (12) aufgenommenen Volumenbildelemente erzeugt werden.

## Claims

1. An apparatus for assisting the positioning and angulation of a manually freely movable tool relative to an object to be treated, the tool having a longitudinal axis, comprising
- a processing unit (20) for the recording and processing of volume image elements of the interior of the object (12),
- a display means (18) for display of the recorded volume image elements,
- a data input means (24) for marking a treatment site (22) within the object (12) and for marking an entry site (26) on the top surface of the object (12) provided for introducing the tool (14) and for allowing the tool (14) to access the treatment site (22), the treatment and entry sites (22,26) being markable and recordable in the processing unit (20) on the basis of the recorded volume image elements of the object (12) which are displayed by the display means (18), and
- a detector (13) for the tool (14), provided for detection of the position of the tool (14) and for angulating the tool (14) relative to the object, and for providing position and angulation data to the processing unit (20), wherein
- the display means (18) is adapted to be controlled by the processing unit (20) in such a manner that
- on the basis of the recorded volume image elements and the recorded entry site, a visual representation can be displayed of the entry site and of that sectional plane of the entry site of the object (12) which is perpendicular to the connecting line (28) between the treatment site (22) and the entry site (26), including the entry site (26),
- and that, in this representation of the sectional plane of the entry site, also a representation of the tool (14) can be displayed, notably as a projection as viewed in the direction of the sectional plane of the entry site and located in front of this plane, so that, on the basis of the representation of the sectional plane of the entry site as a guiding image with the tool position depicted in front of it, the tool (14) can be positioned and orientated until the longitudinal axis of the tool (14) is in alignment with the connecting line (28) between the treatment site (22) and the entry site (26).

2. The apparatus according to claim 1, **characterized in that** the display means (18) is adapted to be controlled by the processing unit (20) to display the object (12) together with the representation of the tool (14) which is generated on the basis of the previously recorded volume image elements and which shows a sectional plane including the connecting line (28) between the treatment site (22) and the entry site (26).

3. The apparatus according to claim 1 or 2, **characterized in that** a means (10) is provided for generating volume image elements of the object (12), and that the display means (18) is adapted to be controlled by the processing unit (20) to additionally display, during the entrance of the tool (14) into the object (12), a representation of sectional planes as a monitoring picture, the sectional planes having predetermined adjustable orientations relative to the tool (14) and being generated on the basis of current volume image elements generated from the object (12).

4. The apparatus according to any one of claims 1 to 3, **characterized in that** the display means (18) is adapted to be controlled by the processing unit (20) to additionally display, during the entrance of the tool (14) into the object (12), a representation of sectional planes as a guiding picture, the sectional planes having predetermined adjustable orientations relative to the tool (14) and being generated on the basis of previous volume image elements generated from the object (12).

## Revendications

1. Dispositif pour assister le positionnement et l'alignement d'un outil présentant un axe longitudinal et pouvant être déplacé manuellement, de façon libre, par rapport à un objet à traiter, comprenant :
- une unité de traitement (20) servant à mémoriser et à traiter des éléments d'image en volume de l'intérieur de l'objet (12),
- un dispositif de visualisation (18) servant à visualiser les éléments d'image en volume mémorisés,
- un dispositif d'entrée de données (24) servant à marquer un point d'intervention (22) à l'intérieur de l'objet (12) et servant à marquer un point d'entrée (26) sur la partie supérieure de l'objet (12) pour introduire l'outil (14), et servant d'accès au point d'intervention (22) par l'outil (14), où le point d'intervention et le point d'entrée (22, 26) peuvent être marqués à l'aide des éléments d'image en volume de l'objet (12) mémorisés, visualisés par le dispositif de visualisation (18), et peuvent être mémorisés dans l'unité de traitement (20), et
- un détecteur (13) pour l'outil (14) servant à détecter la position et l'alignement de l'outil (14) par rapport à l'objet, et servant à fournir à l'unité de traitement (20), des données de positionnement et d'alignement,
- où le dispositif de visualisation (18) peut être commandé par l'unité de traitement (20) de manière telle,
- que, sur la base des éléments d'image en volume mémorisés et du point d'entrée mémorisé, celui-ci ainsi que le plan de coupe du point d'entrée de l'objet (12), qui s'étend perpendiculairement à la ligne de jonction (28) comprise entre le point d'intervention (22) et le point d'entrée (26) et contient le point d'entrée (26), peuvent être représentés,
- et que, dans cette représentation du plan de coupe du point d'entrée, une représentation de l'outil (14) peut être visualisée aussi, en même temps, et ce, comme projection en regardant dans la direction du plan de coupe du point d'entrée et disposée devant ce plan de coupe, de sorte que l'outil (14), à l'aide de la représentation du plan de coupe du point d'entrée servant d'image directrice, associée à une projection de l'outil représentée devant cette image directrice, peut être positionné et aligné aussi longtemps que l'axe longitudinal de l'outil (14) est aligné avec la ligne de jonction (28) comprise entre le point d'intervention (22) et le point d'entrée (26).

2. Dispositif selon la revendication 1, **caractérisé en ce que** le dispositif de visualisation (18) peut être commandé par l'unité de traitement (20) de manière telle, que l'objet (12) peut être représenté par la représentation de l'outil (14) qui est produite à l'aide des éléments d'image en volume enregistrés avant cette représentation et qui montre un plan de coupe contenant la ligne de jonction (28) comprise entre le point d'intervention (22) et le point d'entrée (26).

3. Dispositif selon la revendication 1 ou 2, **caractérisé en ce qu'**il est prévu un dispositif (10) servant à produire des éléments d'image en volume de l'objet (12), et **en ce que** le dispositif de visualisation (18) peut être commandé par l'unité de traitement (20) de manière telle, que lors de la pénétration de l'outil (14) dans l'objet (12), est montrée, en particulier et en plus, une représentation de plans de coupe servant d'image de contrôle, lesquels plans de coupe présentent des alignements prédéterminés réglables par rapport à l'outil (14) et sont produits à l'aide d'éléments d'image en volume enregistrés, de l'objet (12) actuellement.

4. Dispositif selon l'une des revendications 1 à 3, **caractérisé en ce que** le dispositif de visualisation (18) peut être commandé par l'unité de traitement (20) de manière telle, que lors de la pénétration de l'outil (14) dans l'objet (12), est montrée, en particulier et en plus, une représentation de plans de coupe servant d'image directrice, lesquels plans de coupe présentent des alignements prédéterminés réglables par rapport à l'outil (14) et sont produits à l'aide des éléments d'image en volume enregistrés, de l'objet (12) auparavant.
